# EUROPÄISCHE PATENTSCHRIFT

(11) **EP 0 677 286 B1**
(45) Veröffentlichungstag und Bekanntmachung des Hinweises auf die Patenterteilung: **05.08.1998**
(21) Anmeldenummer: 95101099.0
(22) Anmeldetag: 27.01.1995
(51) Int. Cl.: A61K 6/083

(54) **Künstlicher Zahn**
Artificial tooth
Dent artificielle

(30) Priorität: 14.04.1994 DE 4412831
(43) Veröffentlichungstag der Anmeldung: 18.10.1995
(73) Patentinhaber: HERAEUS KULZER GMBH, 63450 Hanau (DE)
(72) Erfinder: Nagel, Joachim, D-61381 Friedrichsdorf (DE); Erdrich, Albert, Dr., D-61231 Bad Nauheim (DE)
(74) Vertreter: Kühn, Hans-Christian

(56) Entgegenhaltungen:
- EP-A- 0 486 775
- WO-A-81/02254
- DE-A- 4 029 230
- US-A- 4 952 530

## Beschreibung

Die Erfindung betrifft einen künstlichen Zahn, der Polymethacrylat, Bariumaluminiumsilicat-Glas und mikrofeines Siliciumdioxid enthält.

Für die Herstellung von Zahnprothesen werden Keramikzähne und Kunststoffzähne verwendet.

Schon lange im Gebrauch sind Kunststoffzähne, die aus Polymethacrylat bestehen und durch Polymerisation von Mischungen aus polymerem und monomerem Methylmethacrylat und gegebenenfalls vernetzend wirkenden Estern der Methacrylsäure mit mehrwertigen Alkoholen nach dem sogenannten Pulver/Flüssigkeits-Verfahren hergestellt werden (R. Marxkors/H. Meiners, Taschenbuch der zahnärztlichen Werkstoffkunde, 1. Auflage, 1978, Carl Hanser Verlag München, 91).

Aus DE 2 312 258 A sind abrasionsfeste und polierbare Werkstoffe und daraus bestehende künstliche Zähne aus einer Polymer-Matrix und darin dispergiertem feinteiligem anorganischem Füllstoff (Makrofüller) bekannt. Der Füllstoff besteht aus Glas und/oder Keramik mit einer Minimalgröße von 0,8 - 8 Mikrometer und einer Maximalgröße von 3 - 20 Mikrometer. Für die Bildung der Polymer-Matrix nach dem Pulver/Flüssigkeits-Verfahren können pulverförmige Methacrylsäureester-Homo- und -Copolymere und flüssige Monomere, wie Methylmethacrylat, Butylacrylat, Polyäthylenglykoldimethacrylat und Bisphenol-A-dimethacrylat, eingesetzt werde.

In DE 24 62 271 C werden dentale Formkörper, besonders künstliche Zähne und Zahnersatzteile, beschrieben, die aus Acrylat- beziehungsweise Methacrylat-Polymeren und mikrofeinem Siliciumdioxid (Mikrofüller) bestehen. Das mikrofeine Siliciumdioxid mit einer Teilchengröße von 10 - 400 Nanometer ist silanisiert, zum Beispiel durch Behandlung mit 3-Methacryloyloxypropyltrimethoxysilan, und kann in einer Menge von 20 - 80 Gewichts-% in den Formkörpern enthalten sein. Es hat sich aber gezeigt, daß mikrofeines Siliciumdioxid enthaltende Kunststoff-Zähne zur Plaque-Anlagerung neigen. Auch sind sie gegenüber Druck-Belastung nicht ausreichend beständig, so daß Verfärbungen und vorzeitige Abrasion verursachende craquelé-artige Risse sich bilden und die Zähne brechen können.

Aus DE 24 05 578 C ist es bekannt, wenigstens die äußere Schicht künstlicher Zähne aus einem Material herzustellen, das neben Estern der Methacrylsäure 30 - 80 Gewichts-% eines anorganischen Füllstoffs aus einer Mischung von durch Flammhydrolyse hergestellter amorpher Kieselsäure mit feinteiligem Glas, zum Beispiel Bariumaluminiumsilicat-Glas, enthält. Der Anteil des Glases kann dabei bis zu 25 Gewichts-% der Füllstoff-Mischung ausmachen.

In DE 38 26 233 C wird ein Verbund-Zahnersatzteil für die Versorgung mit Kronen und Brücken und für Inlays und dergleichen aus einem Kern mit hoher Biegefestigkeit und hohem Biegemodul und abrasionsfestem Mantel mit hochglänzender Oberfläche vorgeschlagen. Kern und Mantel bestehen aus Acrylat- beziehungsweise Methacrylat-Kunststoff und anorganischem Füllstoff, der im Falle des Kerns aus 80 - 90 Gewichts-% Bariumaluminiumsilicat-Glas mit einer mittleren Teilchengröße von 0,7 - 10 Mikrometer und 10 - 20 Gewichts-% hochdispersem Siliciumdioxid mit einer mittleren Teilchengröße von 0,01 - 0,4 Mikrometer bestehen kann. Der Kern enthält 30 - 90 Gewichts-% des Füllstoffs. Der Kunststoff von Kern und Mantel ist vorzugsweise ein Polymer aus Bis-[4-(2-hydroxy-3-methacryloyloxypropoxy)-phenyl]-dimethylmethan (sogenanntes Bis-GMA oder Bowen-Monomer, US 3 066 112), äthoxyliertem Bisphenol A-diacrylat, äthoxyliertem Bisphenol A-dimethacrylat, Triäthylenglykoldimethacrylat, Dodecandioldimethacrylat, Urethandimethacrylat aus 2-Hydroxyäthylmethacrylat und 2,2,4-Trimethylhexamethylendiisocyanat, Bis-(acryloyloxymethyl)-tricyclo[5.2.1.0^{2,6}]decan und/oder Bis-(methacryloyloxymethyl)-tricyclo[5.2.1.0^{2,6}]decan.

DE 40 29 230 A betrifft einen Dentalwerkstoff auf der Basis von polymerisierbaren Monomeren als Bindemittel, der 20 - 90 Gewichts-% einer Füllstoff-Mischung aus amorphen, kugelförmigen Siliciumdioxid-Teilchen und bis zu 20 Mol-% eines Oxids mindestens eines Elements der Gruppen I, II, III und IV mit einer durchschnittlichen Primärteilchengröße von 0,1 - 1,0 Mikrometer, Quarz-, Glaskeramik- oder Glaspulver mit einer durchschnittlichen Teilchengröße von 0,5 - 5,0 Mikrometer und gegebenfalls einen Mikrofüllstoff zur Einstellung der Viskosität enthält. Nach Zusatz von zum Beispiel Dibenzoylperoxid kann daraus ein heißpolymerisierbarer Werkstoff für die Herstellung von Inlays und künstlichen Zähnen erhalten werden. Transparenz und Polierbarkeit werden als sehr gut bezeichnet.

Ein sowohl konventionelle anorganische Füllstoffe (Makrofüller) als auch mikrofeine anorganische Füllstoffe enthaltendes Dentalmaterial - dafür hat sich die Bezeichnung Hybrid-Composite eingebürgert - wird zum Beispiel auch in der internationalen Patentanmeldung WO 81/02 254 beschrieben. Es enthält ein Füllstoff-Gemisch aus hydrophobem Siliciumdioxid mit einem Durchmesser von 0,01 - 0,04 Mikrometer und Glas, zum Beispiel röntgenopakes Barium- oder Strontium-haltiges Glas, mit einem Durchmesser von 2 - 30 Mikrometer. Als polymerisierbare Monomere dienen Bis-GMA oder äthoxyliertes Bisphenol A-dimethacrylat und Triäthylenglykoldimethacrylat. Das Material wird als Zahnfüllmaterial und zum Verblenden von zum Beispiel gegossenen Gold-Kronen verwendet.

Aus DE 41 10 612 A ist ein durch Photopolymerisation auszuhärtendes Dentalmaterial bekannt, das im wesentlichen aus 10 - 60 Gewichts-% Bis-[4-(2-hydroxy-3-methacryloyloxypropoxy)-phenyl]-dimethylmethan, Triäthylenglykoldimethacrylat und/oder dem Diurethandimethacrylat aus 2,2,4-Trimethylhexamethylendiisocyanat und 2-Hydroxyäthylmethacrylat, 37 - 87 Gewichts-% eines Füllstoff-Gemisches aus 80 - 90 Gewichts-% Bariumaluminiumsilicat-Glas mit einer mittleren Teilchengröße von 0,5 - 1,5 Mikrometer und 10 - 20 Gewichts-% mikrofeinem Siliciumdioxid mit einer mittleren Teilchengröße von 0,04 - 0,06 Mikrometer, 0,02 - 2 Gewichts-% α-Diketon und 0,1 - 1 Gewichts-% Amin besteht. Aus dem Dentalmaterial lassen sich röntgenopake, abrasionsfeste und hochglanzpolierbare Zahnfüllungen und Inlays herstellen.

Der Erfindung liegt das Problem zugrunde, einen aus Methacrylat-Kunststoff und anorganischem Füllstoff bestehenden künstlichen Zahn der eingangs charakterisierten Art zu finden, der den natürlichen Zahn in seinen wesentlichen Eigenschaften, nämlich mechanische Festigkeit und Abrasionsverhalten sowie Transparenz und Farbtiefenwirkung, imitiert.

Der die Lösung des Problems darstellende künstliche Zahn ist erfindungsgemäß dadurch gekennzeichnet, daß er im wesentlichen aus 15 - 35 Gewichts-% Polymethacrylat, 35 - 75 Gewichts-% Bariumaluminiumsilicat-Glas mit einer mittleren Teilchengröße von 0,1 - 5,0 Mikrometer und 5 - 25 Gewichts-% Siliciumdioxid mit einer mittleren Teilchengröße von 0,01 - 0,2 Mikrometer besteht.

Besonders bewährt hat sich der im wesentlichen aus 20 - 30 Gewichts-% Polymethacrylat, 50 - 70 Gewichts-% Bariumaluminiumsilicat-Glas mit einer mittleren Teilchengröße von 0,5 - 3 Mikrometer und 5 - 15 Gewichts-% Siliciumdioxid mit einer mittleren Teilchengröße von 0,04 - 0,1 Mikrometer bestehende künstliche Zahn.

Das Bariumaluminiumsilicat-Glas und das Siliciumdioxid sind vorzugsweise silanisiert, zum Beispiel durch Behandlung mit 3-Methacryloyloxypropyltrimethoxysilan.

Außer den genannten Bestandteilen enthält der künstliche Zahn noch eine geringe Menge eines Pigment-Zusatzes.

Das genaue Mischungsverhältnis der den künstlichen Zahn bildenden Bestandteile wird durch Vorversuche auf der Basis der Transparenzeigenschaften des Polymethacrylat-Kunststoffes und der Füllstoffe und der zu erzielenden physikalischen Eigenschaften der Mischung anhand der zur Verfügung stehenden Rohstoffe ermittelt. Hierbei ist es erforderlich, daß der Kunststoff und die Füllstoff-Teilchen einen ähnlichen Brechungsindex haben. Die Bariumaluminiumsilicat-Glas-Teilchen liegen vorzugsweise in einer engen Gauß'schen Größenverteilung zwischen 0,1 und 5 Mikrometer vor.

Als Polymethacrylat haben sich Copolymere aus 30 - 70 Gewichts-%, vorzugsweise 50 - 70 Gewichts-%, Bis[4-(2-hydroxy-3-methacryloyloxypropoxyphenyl)]-dimethylmethan und 30 - 70 Gewichts-%, vorzugsweise 30 - 50 Gewichts-%, Triäthylenglykoldimethacrylat bewährt. Als besonders vorteilhaft hat sich dabei das Copolymer aus einer zu zwei Drittel aus dem ersten Monomer und zu einem Drittel aus dem zweiten Monomer bestehenden Mischung erwiesen.

Der im wesentlichen aus 21 Gewichts-% dieses Copolymers, 70 Gewichts-% Bariumaluminiumsilicat-Glas mit einer mittleren Teilchengröße von 0,1 - 3 Mikrometer und 9 Gewichts-% Siliciumdioxid mit einer mittleren Teilchengröße von 0,04 Mikrometer (Aerosil OX50, Degussa AG) bestehende künstliche Zahn hat sich besonders bewährt.

Charakteristisch für den künstlichen Zahn gemäß der Erfindung ist die ideale Kombination der optischen Eigenschaften - Transparenz und Farbeindruck mit Farbtiefenwirkung - und der physikalischen Eigenschaften, die denen des natürlichen Zahnes sehr nahe kommen. Damit gelingt die Imitation des natürlichen Zahnes.

Der künstliche Zahn gemäß der Erfindung zeichnet sich durch ein sehr günstiges Abrasionsverhalten (7 Mikrometer/Jahr) aus, das dem des natürlichen Schmelzes (8 Mikrometer/Jahr) ähnlich ist. Seine physikalischen Eigenschaften sind besser als die bekannter Kunststoff-Zähne; der Elastizitätsmodul beträgt mehr als 12000 MPa.

Die Herstellung des künstlichen Zahnes gemäß der Erfindung erfolgt in an sich bekannter Weise durch Vermischen der das Polymethacrylat bildenden Monomeren, des Bariumaluminiumsilicat-Glases, des Siliciumdioxids, einer geringen Menge eines Pigment-Zusatzes und eines Katalysators für die Heißpolymerisation, zum Beispiel Dibenzoylperoxid, Einbringen der erhaltenen teigförmigen Mischung in eine Form und Polymerisation der Mischung unter Druck bei Temperaturen zwischen 80 und 170 °C.

Die physikalischen Eigenschaften - Vickershärte, Druckfestigkeit, Biegefestigskeit, Elastizitätsmodul (E-Modul) und Abrasionsfestigkeit, gemessen als Abrasion in Mikrometer/Jahr, - des künstlichen Zahnes gemäß der Erfindung und - zum Vergleich dazu - von handelsüblichen künstlichen Zähnen und von Schmelz und Dentin des natürlichen Zahnes werden in der Tabelle angegeben.

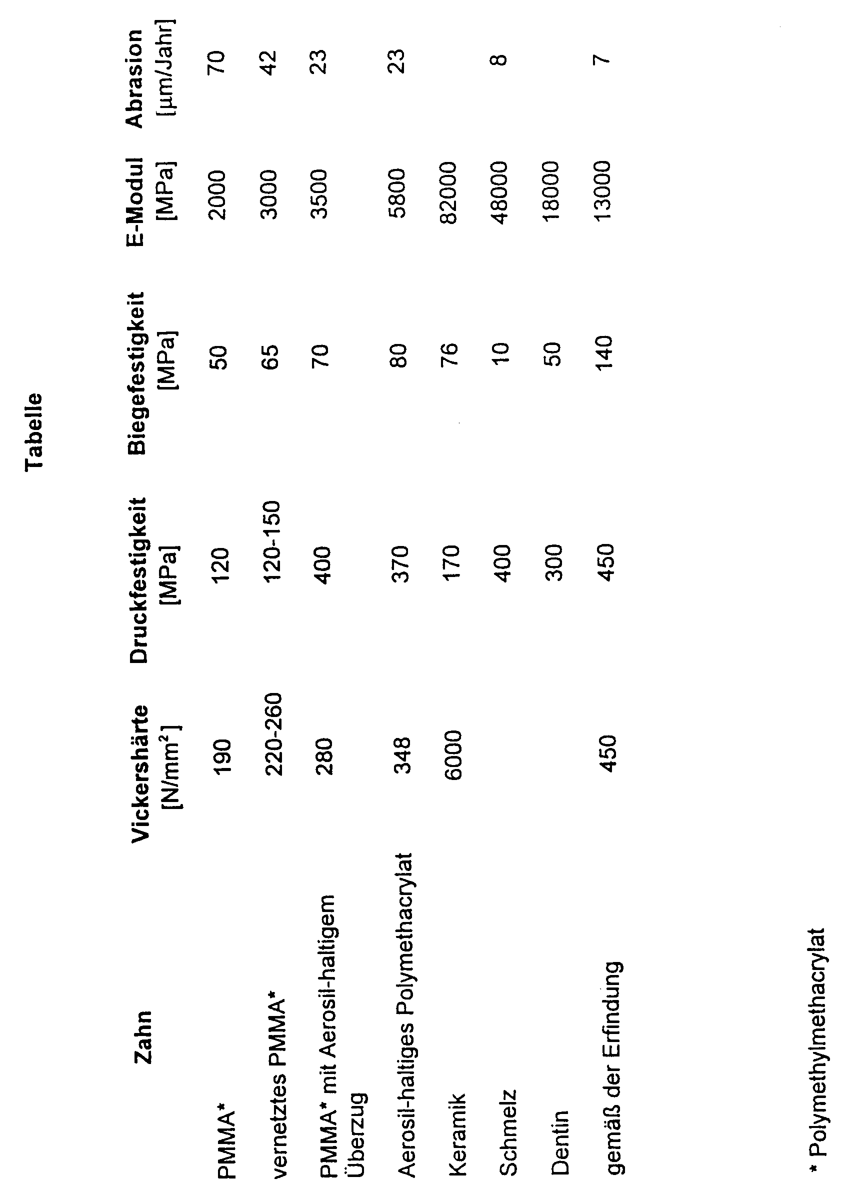

## Patentansprüche

1. Künstlicher Zahn, der Polymethacrylat, Bariumaluminiumsilicat-Glas und mikrofeines Siliciumdioxid enthält, dadurch gekennzeichnet, daß er im wesentlichen aus 15 - 35 Gewichts-% Polymethacrylat, 35 - 75 Gewichts-% Bariumaluminiumsilicat-Glas mit einer mittleren Teilchengröße von 0,1 - 5 Mikrometer und 5 - 25 Gewichts-% Siliciumdioxid mit einer mittleren Teilchengröße von 0,01 - 0,2 Mikrometer besteht.

2. Künstlicher Zahn nach Anspruch 1, dadurch gekennzeichnet, daß er im wesentlichen aus 20 - 30 Gewichts-% Polymethacrylat, 50 - 70 Gewichts-% Bariumaluminiumsilicat-Glas mit einer mittleren Teilchengröße von 0,5 - 3 Mikrometer und 5 - 15 Gewichts-% Siliciumdioxid mit einer mittleren Teilchengröße von 0,04 - 0,1 Mikrometer besteht.

3. Künstlicher Zahn nach Anspruch 1 oder 2, dadurch gekennzeichnet, daß das Polymethacrylat eine Copolymer aus 30 - 70 Gewichts-% Bis-[4-(2-hydroxy-3-methacryloyloxypropoxyphenyl)]-dimethylmethan und 30 - 70 Gewichts-% Triäthylenglykoldimethacrylat ist.

4. Künstlicher Zahn nach Anspruch 3, dadurch gekennzeichnet, daß das Polymethacrylat ein Copolymer aus 50 - 70 Gewichts-% Bis-[4-(2-hydroxy-3-methacryloyloxypropoxyphenyl)]-dimethylmethan und 30 - 50 Gewichts-% Triäthylenglykoldimethacrylat ist.

5. Künstlicher Zahn nach einem der Ansprüche 1 bis 4, dadurch gekennzeichnet, daß das Bariumaluminiumsilicat-Glas und das Siliciumdioxid silanisiert sind.

6. Künstlicher Zahn nach Anspruch 5, dadurch gekennzeichnet, daß das Bariumaluminiumsilicat-Glas und das Siliciumdioxid mit 3-Methacryloyloxypropyltrimethoxysilan silanisiert sind.

## Claims

1. Artificial tooth which contains polymethacrylate, barium aluminium silicate glass and microfine silica, characterized in that it essentially consists of 15 - 35% by weight of polymethacrylate, 35 - 75% by weight of barium aluminium silicate glass having a mean particle size of 0.1 - 5 micrometers and 5 - 25% by weight of silica having a mean particle size of 0.01 - 0.2 micrometer.

2. Artificial tooth according to Claim 1, characterized in that it essentially consists of 20 - 30% by weight of polymethacrylate, 50 - 70% by weight of barium aluminium silicate glass having a mean particle size of 0.5 - 3 micrometers and 5 - 15% by weight of silica having a mean particle size of 0.04 - 0.1 micrometer.

3. Artificial tooth according to Claim 1 or 2, characterized in that the polymethacrylate is a copolymer of 30 - 70% by weight of bis[4-(2-hydroxy-3-methacryloyloxypropoxyphenyl)]dimethylmethane and 30 - 70% by weight of triethylene glycol dimethacrylate.

4. Artificial tooth according to Claim 3, characterized in that the polymethacrylate is a copolymer of 50 - 70% by weight of bis[4-(2-hydroxy-3-methacryloyloxypropoxyphenyl)]dimethylmethane and 30 - 50% by weight of triethylene glycol dimethacrylate.

5. Artificial tooth according to any of Claims 1 to 4, characterized in that the barium aluminium silicate glass and the silica are silanized.

6. Artificial tooth according to Claim 5, characterized in that the barium aluminium silicate glass and the silica are silanized with 3-methacryloyloxypropyltrimethoxysilane.

## Revendications

1. Dent artificielle qui contient du polyméthacrylate, du silicate vitreux de baryum et d'aluminium et du dioxyde de silicium microfin caractérisée par le fait qu'elle est essentiellement constituée de 15-35 % en poids de polyméthacrylate, de 35-75 % en poids d'un silicate vitreux de baryum et d'aluminium d'une dimension moyenne de particules de 0,1-5,0 microns et de 5-25 % en poids de dioxyde de silicium d'une dimension moyenne de particules de 0,01-0,2 microns.

2. Dent artificielle selon là revendication 1, caractérisée par le fait qu'elle est essentiellement constituée de 20-30 % en poids de polyméthacrylate, de 50-70 % en poids d'un silicate vitreux de baryum et d'aluminium d'une dimension moyenne de particules de 0,5-3,0 microns et de 5-15 % en poids de dioxyde de silicium d'une dimension moyenne de particules de 0,04-0,1 microns.

3. Dent artificielle selon la revendication 1 ou 2, caractérisée par le fait que le polyméthacrylate est un copolymère de 30-70 % en poids de bis-[4-(2-hydroxy-3méthacryloyloxypropoxy)-phényl]-diméthylméthane et de 30-70 % en poids de diméthacrylate de triéthylèneglyeol.

4. Dent artificielle selon la revendication 3, caractérisée par le fait que le polyméthacrylate est un copolymère de 50-70 % en poids de bis-[4-(2-hydroxy-3méthacryloyloxypropoxy)-phényl]-diméthylméthane et de 30-50 % en poids de diméthacrylate de triéthylèneglycol.

5. Dent artificielle selon l'une des revendications 1 à 4, caractérisée par le fait que le silicate vitreux de baryum et d'aluminium et le dioxyde de silicium sont silanisés.

6. Dent artificielle selon la revendication 5, caractérisée par le fait que le silicate vitreux de baryum et d'aluminium et le dioxyde de silicium sont silanisés par le 3-méthacryloyloxypropyltriméthoxysilane.
